# EUROPEAN PATENT APPLICATION

(11) **EP 3 677 689 A1**
(43) Date of publication of application: **08.07.2020**
(21) Application number: 18851054.9
(22) Date of filing: 29.08.2018
(51) Int. Cl.: C12Q 1/68, G01N 33/50, G01N 33/68, C12N 15/09

(54) **METHOD FOR DIAGNOSING PREGNANCY IN RUMINANT**

(30) Priority: 29.08.2017 JP 2017163839
(71) Applicant: National University Corporation Hokkaido University, Sapporo-shi, Hokkaido 060-0808 (JP)
(72) Inventor: TAKAHASHI Masashi, Sapporo-shi Hokkaido 060-0808 (JP); KUNII Hiroki, Sapporo-shi Hokkaido 060-0808 (JP); ITO Tsukino, Sapporo-shi Hokkaido 060-0808 (JP); KAWAHARA Manabu, Sapporo-shi Hokkaido 060-0808 (JP); BAI Hanako, Sapporo-shi Hokkaido 060-0808 (JP); SUZUKI, Toshiyuki, Sapporo-shi Hokkaido 060-0808 (JP)
(74) Representative: Wasner, Marita
(86) International application number: PCT/JP2018/031901
(87) International publication number: WO 2019/044885

(57) **Abstract**

[Problem]

To diagnose pregnancy in a livestock animal by a method which can be carried out at a breeding site while minimizing invasion of the subject animal and fetuses thereof.

[Solution]

The method according to the present invention for diagnosing pregnancy in a ruminant comprises: a measurement step of using a sample containing mucosal epithelial cells collected from the genitals ranging from the uterine cervical canal to the vulvar orifice of the subject ruminant, said subject ruminant being in the state corresponding to the early stage of pregnancy, and measuring the expression level of a molecule, which is induced in response to IFN-τ, in the mucosal epithelial cells; and a diagnosis step for comparing the expression level with an expression level of the aforesaid molecule in corresponding mucosal epithelial cells of a non pregnant ruminant of the same species and thus diagnosing pregnancy in the subject ruminant. According to the present invention, pregnancy can be quickly diagnosed before a new ovulation cycle starts after breeding, artificial insemination or fertilized egg transplantation.

## Description

### Field

The present invention relates to a method for determining pregnancy in a ruminant animal.

### Background

In recent years, breeding of domestic animals such as cows, sheep, and goats has been mostly performed through artificial insemination or embryo transfer, regardless of their applications for meat, milk, or the like. However, the conception rate by artificial insemination or embryo transfer is not high, and for example, the conception rate of milk cows is less than 50%. The low conception rate imposes a serious economic burden on livestock raisers such as an increase in the production cost, caused by an increase in the number of artificial inseminations or embryo transfer, an extension of a non-pregnant period, and calving interval, and the like.

It has been pointed out that the low conception rate is caused by weakening of estrus signs in domestic animals, a negative impact of genetic improvement on the breeding traits, large-scale breeding by large-scale farms, or the like. However, the underlying causes are not clear, and although efforts have been made to increase the conception rate, there is no sign of improvement. Thus, it has been strongly desired to determine pregnancy at an early stage in domestic animals that have undergone artificial insemination or embryo transfer, and shorten the non-pregnant period and calving interval.

Pregnancy in cows is mainly determined by a rectal examination method and an ultrasonic diagnosis method. Determination results of both methods are reliable, but both methods need to be carried out after a month or more after fertilization. Because the ovulation cycle of cows is 21 days, the waiting of a month or more described above means that a chance of performing artificial insemination or embryo transfer anew is lost once or twice, until it is determined that the cow is not pregnant. Consequently, during the non-pregnant period, which occurs as the result, there will be no production income, and loss of the breeding expenses will increase.

In such a case, if it is possible to quickly determine pregnancy after artificial insemination or embryo transfer, and before the ovulation cycle ends, it is possible to perform another artificial insemination or embryo transfer into a non-conceived cow according to the next ovulation cycle. Consequently, it is expected to improve the actual conception rate.

In ruminant animals, interferon-tau (IFN-τ), which is one of type-I interferons, is responsible for implantation of a fertilized embryo. Taking a cow as an example, an embryo that has entered the uterus with repeating cleavages, reaches the blastocyst stage during which the embryo is differentiated into two types of cell groups of an inner cell mass that can become a fetus in future and trophoblast cells that form placenta. IFN-τ is produced from trophoblast cells, thereby the mother's body recognizes the presence of the embryo, and regulates the intrauterine environment suitable for embryo implantation.

It has been generally known that there is a common pattern in the production profile of IFN-τ from trophoblast cells of ruminant animals, regardless of the type of ruminant animals. Specifically, a small amount of IFN-τ is produced in a period from the cleavage to blastocyst. The production of IFN-τ is rapidly increased after hatching which the embryo that has reached the uterus escaped from zona pellucida, a glycoprotein capsule surrounding embryo. The production of IFN-τ reaches a peak before implantation, and rapidly decreases after implantation. It has also been known that in maternal uterine endometrial tissue, the expression of an IFN-τ inducible factor (interferon stimulated genes, ISGs) such as ISG15, Mx1, Mx2, and OAS-1 is induced by the stimulation of IFN-τ produced from trophoblast cells. On the other hand, when an egg is not fertilized, or when a fertilized embryo is not present in the uterus even if an egg is fertilized, pregnancy is not established. Thus, the production of IFN-τ or the expression of the IFN-τ inducible factor will not be confirmed, and a new ovulation cycle starts.

From the mechanism described above, it is expected that the production of IFN-τ by the conceptus in the uterine cavity, and the expression of the IFN-τ inducible factor in the endometrial tissue will be indicators for determining pregnancy at an early stage in ruminant animals. When tissue in the uterus is collected as a sample, there may be an invasive impact on the target animal and an influence on the fetus. Thus, a pregnancy determination method by detecting the expression of the IFN-τ inducible factor using the mother's blood sample (Patent Literature 1) has been developed. However, the reliability of determination results of blood diagnosis is not necessarily high, because the gene expression level varies for each individual and the like.

### Citation List

### Patent Literature

Patent Literature 1: Japanese Unexamined Patent Application Publication No. 2004-534224

### Summary

### Technical Problem

An object of the present invention is to determine pregnancy in domestic animals with little invasive impact on the target animal, with little influence on the fetus, and that can be carried out at breeding sites.

### Solution to Problem

The inventors of the present invention have found out that the IFN-τ inducible factor is expressed in the mucous membranes of the uterine cervical canal and the vaginal wall at the periphery of the external os of uterus that are extrauterine tissues of a pregnant cow, and that the IFN-τ inducible factor is also expressed in the mucous membrane of the vaginal vestibule. Consequently, the inventors have completed the following invention.

(1) A method for determining pregnancy in a ruminant animal including a measurement step of measuring an expression level of molecules induced in response to IFN-τ in mucosal epithelial cells, using a sample containing the mucosal epithelial cells, the sample being collected from the genital organs of a test ruminant animal ranging from the uterine cervical canal to the vulva in a period corresponding to the early stage of pregnancy; and a determination step of determining pregnancy in the test ruminant animal by comparing the expression level with an expression level of the molecules in corresponding mucosal epithelial cells of a non-pregnant ruminant animal of the same species.
(2) The method according to (1), in which the sample containing the mucosal epithelial cells is collected from the genital organs ranging from the vagina to the vulva.
(3) The method according to (1) or (2), in which the sample containing the mucosal epithelial cells is collected from the surface of the genital organs in a low-invasive manner or in a non-invasive manner.
(4) The method according to any one of (1) to (3), in which the sample containing the mucosal epithelial cells does not substantially contain blood.
(5) The method according to any one of (1) to (4), in which each of the molecules induced in response to IFN-τ is mRNA transcribed from at least one gene selected from the group consisting of LOC100139670, ISG15, OAS-1X, OAS-1Y, OAS-1Z, OAS-2, UBA7, USP18, Mx1, Mx2, RSAD2, GBP4, GBP5, TNFSF10, STAT1, STAT2, and PLAC8 genes; or protein encoded from the gene.
(6) The method according to any one of (1) to (5), in which each of the molecules induced in response to IFN-τ is mRNA transcribed from at least one gene selected from the group consisting of ISG15, OAS-1X, Mx1, and Mx2 genes.
(7) The method according to any one of (1) to (6), in which the expression level of the molecules induced in response to IFN-τ is measured by a quantitative PCR method or a LAMP (loop-mediated isothermal amplification) method.
(8) The method according to any one of (1) to (7), in which the expression level of the molecules induced in response to IFN-τ is measured without separation and purification of RNA from the mucosal epithelial cells.
(9) The method according to any one of (1) to (8), in which the ruminant animal is selected from the group consisting of cow, sheep, goat, water buffalo, yak, and deer.
(10) A kit for determining pregnancy in a ruminant animal, the kit including a reagent to measure an expression level of molecules induced in response to IFN-τ in mucosal epithelial cells of a test ruminant animal, and an instrument to collect a sample containing the cells.
(11) The kit according to (10), in which each of the molecules induced in response to IFN-τ is mRNA transcribed from at least one gene selected from the group consisting of LOC100139670, ISG15, OAS-1X, OAS-1Y, OAS-1Z, OAS-2, UBA7, USP18, Mx1, Mx2, RSAD2, GBP4, GBP5, TNFSF10, STAT1, STAT2, and PLAC8 genes; or protein encoded from the gene.
(12) The kit according to (10) or (11), in which each of the molecules induced in response to IFN-τ is mRNA transcribed from at least one gene selected from the group consisting of ISG15, OAS-1X, Mx1, and Mx2 genes.
(13) The kit according to any one of (10) to (12), in which each of the molecules induced in response to IFN-τ is mRNA, and the reagent to measure the expression level of the molecules is a reagent for a nucleic acid amplification method.
(14) The kit according to any one of (10) to (13), in which the instrument to collect a sample containing the cells is an instrument to collect a sample in a low-invasive manner or in a non-invasive manner.

### Advantageous Effects of Invention

With the present invention, it is possible to quickly determine pregnancy before a next ovulation cycle starts after mating, artificial insemination, or embryo transfer is carried out. When a ruminant animal has not conceived, it is possible to shorten the non-pregnant period and calving interval, by performing mating, artificial insemination, or embryo transfer according to the next ovulation cycle. Consequently, it is possible to substantially improve the conception rate.

### Brief Description of Drawings

Fig. 1 is a graph indicating relative gene expression levels of ISG15, Mx1, Mx2, and OAS-1X in the mucous membranes of the uterine cervical canal in pregnant cows and non-pregnant cows.
Fig. 2 is a graph indicating relative gene expression levels of ISG15, Mx1, and Mx2 in the mucous membranes at the periphery of the external os in pregnant cows and non-pregnant cows.
Fig. 3 is a graph indicating relative gene expression levels of ISG15 in the mucous membranes of the uterine cervical canal, the periphery of the external os, and vaginal vestibule in pregnant cows and the uterine cervical canal in non-pregnant cows. Fig. 4 is a graph indicating results obtained by quantitatively measuring gene expression levels of ISG15 in the mucous membranes of the uterine cervical canal in pregnant cows and non-pregnant cows by the RT-LAMP method (temporal transition in turbidity).
Fig. 5 is a graph indicating results obtained by quantitatively measuring the gene expression levels of ISG15 in the mucous membranes of the uterine cervical canal in pregnant cows and non-pregnant cows by the RT-LAMP method (turbidity at 30 minutes after the start of LAMP reaction).

### Description of Embodiments

A first aspect of the present invention relates to a method for determining pregnancy in a ruminant animal including a measurement step of measuring the expression level of molecules induced in response to IFN-τ in mucosal epithelial cells, using a sample containing the mucosal epithelial cells, the sample being collected from the genital organs of a test ruminant animal ranging from the uterine cervical canal to the vulva in a period corresponding to the early stage of pregnancy; and a determination step of determining pregnancy in the test ruminant animal by comparing the expression level with an expression level of the molecules in the corresponding mucosal epithelial cells of a non-pregnant ruminant animal of the same species.

The first aspect of the present invention includes the measurement step of measuring the expression level of the molecules induced in response to IFN-τ in mucosal epithelial cells, using the sample containing the mucosal epithelial cells, the sample being collected from the genital organs of a test ruminant animal ranging from the uterine cervical canal to the vulva in a period corresponding to the early stage of pregnancy.

The first aspect of the present invention is applied to a ruminant animal in a period corresponding to the early stage of pregnancy. The test ruminant animal in the period corresponding to the early stage of pregnancy is a ruminant animal that has mated or undergone artificial insemination or embryo transfer in advance. The mating is particularly carried out by what is called natural mating that is copulation with a male of the same species. However, the mating may also be carried out artificially. Moreover, the artificial insemination and the embryo transfer may be carried out by any method known to those skilled in the art, and the application of the present invention is not limited to a specific technique, method, or the like.

The ruminant animal to which the present invention is applied is not particularly limited as long as the animal is a mammal that does rumination. Preferable examples of the ruminant animal include cow, sheep, goat, yak, water buffalo, and deer. More preferable application targets include cow, sheep, goat, and water buffalo. Particularly preferable application targets include cow, water buffalo, and yak. The cow may be beef cow or dairy cow. The water buffalo may be swamp buffalo or river buffalo.

The period corresponding to the early stage of pregnancy is a period from the initiation of pregnancy recognition that is when the fertilized embryo starts producing IFN-τ, until implantation. The period corresponding to the early stage of pregnancy is 14 to 20 days after fertilization in cows, 14 to 20 days after fertilization in sheep, 16 to 20 days after fertilization in goats, 14 to 19 days after fertilization in yaks, 14 to 20 days after fertilization in water buffalo, and 14 to 20 days after fertilization in deer. It is particularly preferable to apply the present invention to a ruminant animal while the ruminant animal is producing IFN-τ close to its peak in the period corresponding to the early stage of pregnancy described above.

When the present invention is applied to a ruminant animal that has mated or undergone artificial insemination, the period corresponding to the early stage of pregnancy is calculated by setting the day when the mating or artificial insemination is carried out as day zero after fertilization. Moreover, when the present invention is applied to a ruminant animal that has undergone embryo transfer, the period corresponding to the early stage of pregnancy is calculated by setting the day when an embryo to be transplanted is fertilized as day zero after fertilization. For example, when an embryo on day seven after fertilization that is collected after seven days from when artificial insemination is carried out to a donor cow is transferred to a recipient cow, the period corresponding to the early stage of pregnancy in the recipient cow is 14 to 20 days after fertilization, in other words, 7 to 13 days after the embryo is transferred.

The genital organs ranging from the uterine cervical canal to the vulva is a portion from the uterine cervical canal reaching to the vulva of the genital organs of a female ruminant animal. The genital organs ranging from the uterine cervical canal to the vulva typically indicate uterine cervical canal, vagina, vaginal vestibule, and vulva. A deep portion of the vagina, in other words, the peripheral portion of the introitus at the vagina side of the uterine cervical canal is also referred to as the periphery of the external os of uterus.

The sample containing the mucosal epithelial cells may be collected from at least one site of the genital organs ranging from the uterine cervical canal to the vulva. The vagina is enlarged directly or by using a speculum as needed. The sample is then collected by inserting an instrument such as a spoon, a cotton swab, an earpick, or sponge, which is suitable for collecting a sample in a low-invasive manner or in a non-invasive manner, until the instrument reaches a desirable collecting site, and by scraping off or wiping off the surface of the sites. The sample containing the collected mucosal epithelial cells may also contain mucus, cells of lamina propria or muscularis mucosae, as long as the sample contains mucosal epithelial cells. Preferably, the sample containing the mucosal epithelial cells is collected from the genital organs ranging from the vagina to the vulva. Preferably, the sample containing the mucosal epithelial cells is collected from the surface of the genital organs in a low-invasive manner or in a non-invasive manner. In a particularly preferable embodiment, the sample containing the mucosal epithelial cells is collected from the surface of the genital organs ranging from the vagina to the vulva in a non-invasive manner, and does not substantially contain blood. The phrase "does not substantially contain blood" means that the sample does not contain an amount of blood that will affect the measurement results when the expression level of the molecules induced in response to IFN-τ is measured using the sample. Typically, majority of the cells contained in the sample that "does not substantially contain blood" are mucosal epithelial cells. For example, the cells contain 90% or more of mucosal epithelial cells, more preferably contains 95% or more of mucosal epithelial cells, further preferably contains 98% or more of mucosal epithelial cells, and still further preferably contains 99% or more of mucosal epithelial cells.

The sample containing the mucosal epithelial cells may be used in the measurement as the sample is collected from the test ruminant animal, or may be used in the measurement after separating the cells contained in the sample. Alternatively, homogenate of the cells may be used in the measurement, or the sample may be used in the measurement after separating components such as mRNA and protein to be measured from them. Moreover, the sample may be used in the measurement after being refrigerated, frozen, or dried, or after being treated by a general storage method such as formalin fixation.

When tissue or cells are directly used in the measurement without separating the components to be measured, it is possible to promote the permeation of the reagent for detecting mRNA or protein into the cells, by performing treatment for enhancing the cell membrane permeability so that the components in the tissue or cells can be easily detected.

The molecules induced in response to IFN-τ include mRNA transcribed from a gene of an IFN-τ inducible factor, and/or protein encoded by the gene. Known IFN-τ inducible factors include ISG15, Mx1, Mx2, OAS-1, OAS-2, LOC100139670, USP18, RSAD2, UBA7, GBP4, GBP5, TNFSF10, JAK1, STAT2, and PLAC8. Moreover, the molecules induced in response to IFN-τ may also be specified by separating mucosal epithelial cells from at least one site, resected from the target ruminant animal, of the genital organs ranging from the uterine cervical canal to the vulva, and by comparing the gene expression profile of the cells cultured in the presence of IFN-τ and that of the cells cultured in the absence of IFN-τ. The gene expression analysis can be performed by a method known to those skilled in the art using a DNA chip, a Gene chip, a microchip, a bead array, or the like.

A preferable example of the molecules induced in response to IFN-τ includes mRNA transcribed from at least one gene selected from the group of genes including LOC100139670, ISG15, OAS-1X, OAS-1Y, OAS-1Z, OAS-2, UBA7, USP18, Mx1, Mx2, RSAD2, GBP4, GBP5, TNFSF10, STAT1, STAT2, and PLAC8; or protein encoded from the gene.

Base sequences of mRNA transcribed from the genes of LOC100139670, ISG15, OAS-1X, OAS-1Y, OAS-1Z, OAS-2, UBA7, USP18, Mx1, Mx2, RSAD2, GBP4, GBP5, TNFSF10, STAT1, STAT2, and PLAC8 of a cow (Bos taurus), and amino acid sequences of the proteins encoded from the genes are registered in GenBank under the accession numbers indicated in Table 1.

**Table 1**

| Gene | mRNA | Protein name | Amino acid sequence |
|---|---|---|---|
| *LOC100139670* | XM_015469501 | interferon-induced protein with tetratricopeptide repeats 1 | XP_015324987 |
| *ISG15* | NM_174366.1 | ubiquitin-like protein | NP_776791 |
| *OAS-1X* | NM_178108 | 2',5'-oligoadenylate synthetase 1 | NP_835209 |
| | XM_597752 | | XP_597752 |
| *OAS-1Y* | NM_001040606 | 2',5'-oligoadenylate synthetase 1 | NP_001035696 |
| *OAS-1Z* | NM_001029846 | 2' ,5'-oligoadenylate synthetase | NP_001025017.1 |
| *OAS-2* | NM_001024557 | 2'-5'-oligoadenylate synthetase 2 | NP_001019728.1 |
| | XM_608114 | | |
| *UBA7* | NM_001012284 | ubiquitin-like modifier activating enzyme 7 | NP_001012284.1 |
| *USP18* | NM_001017940 | ubiquitin specific peptidase 18 | AAI49488 |
| | XM_593181 | | |
| *Mx1* | NM_173940.2 | interferon-induced GTP-binding protein Mx1 | NP_776365 |
| *Mx2* | NM_173941.2 | interferon-induced GTP-binding protein Mx2 | NP_776366 |
| *RSAD2* | NM_001045941 | radical S-adenosyl methionine domain containing 2 | NP_001039406.1 |
| *GBP4* | NM_001102261 | guanylate binding protein 4 | NP_001095731.1 |
| *GBP5* | NM_001075746 | guanylate binding protein 5 | N P_001069214.1 |
| *TNFSF10* | XM_005195915 | tumor necrosis factor (ligand) superfamily, member 10 | XP_005195972.1 |
| *STAT1* | NM_001077900 | signal transducer and activator of transcription1 | NP_001071368.1 |
| *STAT2* | NM_001205689 | signal transducer and activator of transcription 2 | NP_001192618.1 |
| *PLAC8* | NM_001025325.2 | Placenta-specific 8 | NP _001020496 |

The expression level of mRNA can be measured using a known method that can be used in measuring the expression level of mRNA. The method includes a nucleic acid amplification method such as the quantitative polymerase chain reaction (PCR) method or the loop-mediated isothermal amplification (LAMP) method with primer nucleic acids having a suitable base sequence designed on the basis of the base sequence of the target mRNA; a hybridization method with probe nucleic acids having a base sequence capable of hybridizing to the base sequence of mRNA under stringent conditions; and a microarray method with a DNA chip, a Gene chip, a microchip, a bead array, or the like on which nucleic acids having a base sequence capable of hybridizing to the base sequence of mRNA are fixed. The nucleic acids described above may be labeled by a labelling compound such as a fluorescent substance, a radioisotope, an enzyme, biotin, and streptavidin, according to the method used. In particular, when the expression level of mRNA is measured at the breeding sites of ruminant animals, or what is called on-site, the expression level is preferably measured by the quantitative PCR method or the LAMP method.

The expression level of protein can be measured by a known method such as a direct competitive method, an indirect competitive method, an enzyme-linked immunosorbent assay (ELISA) method such as a sandwich method, a radioimmunoassay (RIA) method, in situ hybridization, immunoblot analysis, Western blot analysis, and tissue array analysis, using an antibody specific for target protein. In this case, the specific antibody is not restricted to the species of animal from which the antibody is derived. The specific antibody may be either a polyclonal antibody or a monoclonal antibody. The specific antibody may be an antibody including full-length immunoglobulin, fragments such as Fab fragments and F(ab')2 fragments, or the like.

The specific antibody may also be labeled by a labelling compound such as fluorescent substances (for example, fluorescein isothiocyanate (FITC), rhodamine, and phalloidin), colloidal particles such as gold, fluorescent microbeads such as Luminex (registered trademark, Luminex Corporation), heavy metals (for example, gold and platinum), chromoproteins (for example, phycoerythrin and phycocyanin), radioisotopes (for example, ³H, ¹⁴C, ³²P, ³⁵S, ¹²⁵I, and ¹³¹I), enzymes (for example, peroxidase and alkaline phosphatase), biotin, and streptavidin.

The first aspect of the present invention includes the determination step of determining pregnancy in the test ruminant animal by comparing the expression level of the molecules induced in response to IFN-τ measured in the measurement step described above, with the expression level of the molecules in the corresponding mucosal epithelial cells of a non-pregnant ruminant animal of the same species.

The non-pregnant ruminant animal of the same species refers to a female ruminant animal that has not mated or undergone artificial insemination or embryo transfer, of the same species with the test ruminant animal. Moreover, the corresponding mucosal epithelial cells of the non-pregnant ruminant animal of the same species refer to the mucosal epithelial cells, collected from the same site where the mucosal epithelial cells of the test ruminant animal were collected, of the non-pregnant ruminant animal of the same species. For example, when the mucosal epithelial cells of the test ruminant animal are collected from the uterine cervical canal, the corresponding mucosal epithelial cells of the non-pregnant ruminant animal of the same species are collected from the uterine cervical canal in the non-pregnant ruminant animal of the same species.

The expression level of the molecules in the corresponding mucosal epithelial cells of the non-pregnant ruminant animal of the same species is used as a control for the expression level of the molecules in the test ruminant animal. Consequently, for example, when the expression level of ISG15 mRNA in the mucosal epithelial cells of the uterine cervical canal in a dairy cow on day 18 after fertilization is measured, the expression level of ISG15 mRNA in the mucosal epithelial cells of the uterine cervical canal in a non-pregnant milk cow in the same ovulation cycle will be a control. The control may also be prepared in advance by measuring the expression level of the molecules induced in response to IFN-τ in the period corresponding to the early stage of pregnancy in the non-pregnant ruminant animal. Conditions such as the type of ruminant animal, the collection site of the mucosal epithelial cells, the collection time, molecules to be measured, the measurement method, and the like of the control are as described in the measurement step. Preferably, the same conditions as those in the test ruminant animal may be used.

The expression levels of the molecules induced in response to IFN-τ can be compared in an appropriate manner according to the measurement method. For example, when a real-time PCR method is used to measure the expression level of the molecules, the comparison can be made as follows: A Ct value of a target gene (gene of the molecule described above) and a Ct value of a reference gene (for example, housekeeping gene) are measured, using samples containing the mucosal epithelial cells of the test ruminant animal and the non-pregnant ruminant animal. Then, how many times the expression level of the molecules in the test ruminant animal is higher than that of the molecules in the control is calculated using a comparative Ct method. For example, when a ΔCt value or a ΔTt value of the molecules in the test ruminant animal measured by the real-time PCR method or the LAMP method exceeds the ΔCt value or the ΔTt value of the control by twice or more, preferably by three times or more, more preferably by five times or more, and particularly preferably by 10 times or more, it is possible to determine that the test ruminant animal is pregnant.

The determination made in the determination step may be made by measuring the expression levels of a test ruminant animal and a non-pregnant animal, and directly comparing the expression levels with each other. The determination may also be made by setting the measurement conditions such that the specimen derived from a pregnant animal will be positive, but the specimen derived from a non-pregnant animal will be negative, and observing whether the specimen derived from a test ruminant animal indicates a positive or negative determination result. For example, when the expression level of the molecules is measured by the nucleic acid amplification method such as the PCR method or the LAMP method, the determination may also be made by performing a nucleic acid amplification reaction with diluting the specimens or adjusting the number of cycles or the reaction time of the amplification reaction in an appropriate manner such that the amplification product in the specimen derived from a pregnant animal is detectable while that in the specimen derived from a non-pregnant animal is undetectable or is equal to or less than a predetermined value, and observing the presence of the amplification product or comparing the amount of the amplification product with the predetermined value.

The determination step of the first aspect can be replaced with another determination step of determining pregnancy in the test ruminant animal by comparing the expression level of the molecules induced in response to IFN-τ measured in the measurement step with a cut-off value. Consequently, another aspect of the present invention relates to the method for determining pregnancy in a ruminant animal including the measurement step of measuring the expression level of molecules induced in response to IFN-τ in mucosal epithelial cells, using a sample containing the mucosal epithelial cells, the sample being collected from the genital organs of a test ruminant animal ranging from the uterine cervical canal to the vulva in a period corresponding to the early stage of pregnancy; and the determination step of determining pregnancy in the test ruminant animal by comparing the expression level with the cut-off value.

The cut-off value is set in an appropriate manner using a receiver operating characteristic (ROC) curve, according to the collection site of the sample and the molecules to be measured. When the expression level of the molecules in the test ruminant animal is greater than the cut-off value, it can be determined that the test ruminant animal is pregnant. When the expression level of the molecules in the test ruminant animal is smaller than the cut-off value, it can be determined that the test ruminant animal is not pregnant.

The test ruminant animal determined as non-pregnant on the basis of the criteria described above can be mated, artificially inseminated, or can have an embryo transplanted again, according to the nearest ovulation cycle.

Another aspect of the present invention relates to a kit for determining pregnancy on the basis of the first aspect of the present invention. The kit includes a reagent to measure the expression level of the molecules induced in response to IFN-τ in the mucosal epithelial cells, more specifically, a reagent for carrying out the various measurement methods described above. For example, the reagent includes a reagent for the nucleic acid amplification method or the ELISA method, typically, primer nucleic acid, probe nucleic acid, deoxynucleotide triphosphate (dNTP), a specific antibody, a secondary antibody, an enzyme, a buffer, and a coloring reagent. The kit may also include a device for measuring the expression level of the molecules; an instrument such as a spoon, an earpick, a cotton swab, or sponge for collecting a sample containing the mucosal epithelial cells from the test ruminant animal; and a container or a support for holding the sample. Preferably, the kit may also include an instruction for determining pregnancy in a test ruminant animal on the basis of the expression level of the molecules induced in response to IFN-τ.

The present invention will now be described in more detail with reference to the following Examples. However, the present invention is not limited to the examples.

### Examples

Example 1. Expression Analysis of Interferon Inducible Factors in Extrauterine Tissues (Uterine Cervical canals and Peripheral Portions of External Os of Uterus) of Pregnant Cows and Non-Pregnant Cows

### 1) Collection of Mucous Membranes

Among lactating Holstein cows maintained in the Dairy Research Institute, Experiment Farm, Field Science Center for Northern Biosphere, Hokkaido University, cows on day 18 after artificial insemination the pregnancy of which was confirmed at the later pregnancy diagnosis, and cows those have not undergone artificial insemination were selected, and the vulvas were wiped with invert soap solution. The vaginal vestibule of each cow was opened directly or using a vaginal speculum, and the mucous membranes were scraped and collected from the uterine cervical canal and the periphery of the external os of uterus respectively, with a commercially available measuring spoon inserted into the cow. In visual observation, the collected samples were confirmed not to be contaminated with blood. In microscopy, it was confirmed that most cells (95% or more) in the samples were mucosal epithelial cells. The samples were immediately placed into ice-cooled 1.5 mL tubes filled with 500 µL of ISOGEN II (Nippon Gene), and the tubes were frozen and stored at -80 degrees Celsius.

### 2) Extraction of Total RNA

The frozen mucous membranes were thawed, and added with 500 µL of ISOGEN II, and mixed. RNase free H₂O (Nippon Gene) in an amount of 200 µL was added, and the tubes were shaken well for 15 seconds. After being left stand for 15 minutes at room temperature, the tubes were centrifuged at 12,000 g for 15 minutes at 4 degrees Celsius, and the supernatants were collected in 1.5 mL tubes. To each of the tubes, 2-propanol (Wako) as much as that of the collected supernatant was added, and the tubes were inverted, mixed, and left to stand for 10 minutes at -30 degrees Celsius. After being left to stand, the tubes were centrifuged at 12,000 g for 10 minutes at 4 degrees Celsius, and the supernatants were removed therefrom. Then, 500 µL of 75% ethanol was added to each of the tubes, and the tubes were inverted, mixed, and centrifuged at 15,000 g for 5 minutes at 4 degrees Celsius. The total RNAs were obtained by repeating the process described above, vacuum drying for 10 minutes after removing the supernatants, and adding 30 µL of RNase free H₂O. After measuring the concentration of RNA, the total RNAs were stored at -80 degrees Celsius until use.

### 3) cDNA Synthesis by Reverse Transcription

cDNA reverse transcription from the total RNAs was performed using ReverTra Ace (registered trademark) q-PCR RT Master Mix with gDNA remover (TOYOBO). The processes of heating and cooling required for the reaction were all performed using ASTEC Program Temp Control System PC-815. The total RNA obtained from each of the samples and RNase free H₂O were prepared so that the concentration of the total RNA becomes 100 ng/12 µL. The total RNA and RNase free H₂O were then heat denatured for five minutes at 65 degrees Celsius, and were immediately cooled on ice. Then, to digest genomic DNA, 4 µL of a mixture of 4 × DN Master Mix and gDNA remover at 50 to 1 was added to the tubes, and the tubes were heated for five minutes at 37 degrees Celsius, and immediately cooled on ice. Next, 4 µL of 5 × RT Master Mix II was added to the tubes, and reverse transcription was performed by heating the tubes for 15 minutes at 37 degrees Celsius and for 15 minutes at 50 degrees Celsius, and then enzyme deactivation was performed by heating the tubes for five minutes at 98 degrees Celsius to synthesize cDNAs. The obtained cDNAs were diluted five times by adding 80 µL of sterile water and stored at -30 degrees Celsius until use.

### 4) Realtime PCR

ISG15, Mx1, Mx2, and OAS-1X were selected as genes of interest for evaluating the responsiveness of pregnancy in the extrauterine tissues, and H2AFZ was selected as an internal standard gene. With reference to the base sequences of genes published in the database (NCBI; http://www.ncbi.nlm.nih.gov/), forward and reverse primers (Table 2) were designed and synthesized so that an amplification product of around 150 bp including the exonintron boundary can be obtained.

**Table 2**

| Gene | | Sequence (5'-3') | | Accession No. of reference sequence | Product length (bp) |
|---|---|---|---|---|---|
| *MX1* | F | GCCAACTAGTCAGCACTACATTGTC | (SEQ ID NO.1) | NM_173940.2 | 139 |
| | R | GCTCTTGGACTCCATATCTTCAC | (SEQ ID NO.2) | | |
| *MX2* | F | CAGAGACGCCTCAGTCGAAG | (SEQ ID NO.3) | NM_173941.2 | 113 |
| | R | GAGACGTTTGCTGGTTTCCATG | (SEQ ID NO.4) | | |
| *ISG15* | F | TGAGGGACTCCATGACGGTA | (SEQ ID NO.5) | NM_174366.1 | 72 |
| | R | GCTGGAAAGCAGGCACATTG | (SEQ ID NO.6) | | |
| *OAS1X* | F | TCTGAGGTCCAGAAACGGCA | (SEQ ID NO.7) | NM_178108.2 | 164 |
| | R | AGCTTCACGTAGATTTGAGGGTC | (SEQ ID NO.8) | | |
| *H2AFZ* | F | AGAGCCGGTTTGCAGTTCCCG | (SEQ ID NO.9) | NM_174809.2 | 116 |
| | R | TACTCCAGGATGGCTGCGCTGT | (SEQ ID NO.10) | | |

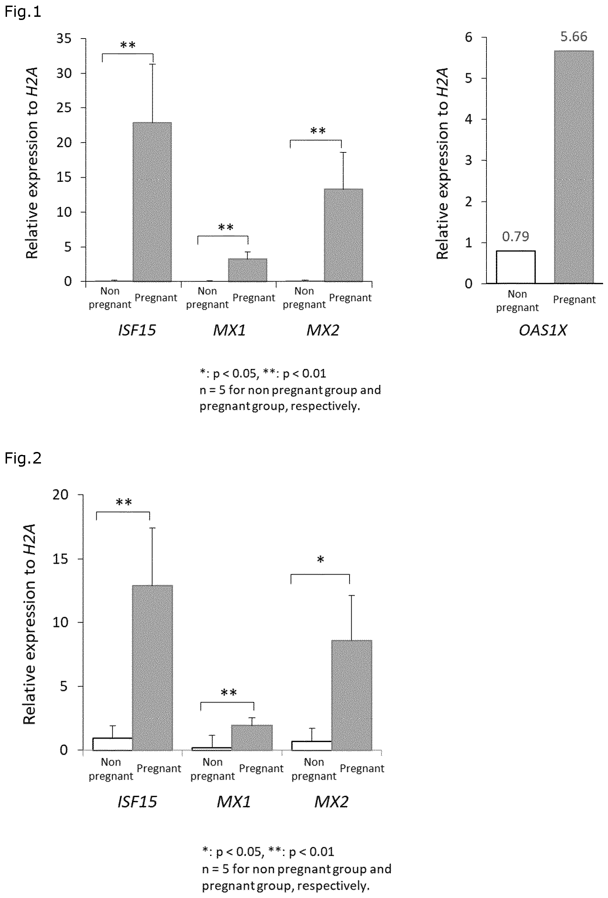

By adding 5 µL of THUNDERBIRD SYBR qPCR Mix (TOYOBO), 0.5 µL each of the forward and reverse primers described above, and 4 µL of the cDNA solution serving as a template, 10 µL of PCR reaction solution was obtained. By using LightCycler Nano (Roche Diagnostics), the real-time PCR was performed as follows: one cycle for 30 seconds at 95 degrees Celsius; 45 cycles for 10 seconds at 95 degrees Celsius, for 15 seconds at 55 degrees Celsius, and for 30 seconds at 72 degrees Celsius; and one cycle each for 20 seconds at 60 degrees Celsius and for 20 seconds at 95 degrees Celsius. Fig. 1 illustrates the relative gene expression levels of ISG15, Mx1, Mx2, and OAS-1X in the uterine cervical canal. Fig. 2 illustrates the relative gene expression levels of ISG15, Mx1, and Mx2 at the periphery of the external os of uterus.

It was confirmed that compared with the expression levels of genes in the mucous membranes of the uterine cervical canal and the periphery of the external os in the non-pregnant cows, the expression level of ISG15 in the mucous membrane of the uterine cervical canal in the pregnancy-confirmed-cows was increased by 122 times, that of Mx1 was increased by 31 times, that of Mx2 was increased by 66 times, and that of OAS-1X was increased by seven times; and the expression level of ISG15 in the mucous membrane at the periphery of the external os in the pregnancy-confirmed-cows was increased by 14 times, that of Mx1 was enhanced by 10 times, and that of Mx2 was increased by 12 times. Consequently, sensitization action by IFN-τ was confirmed in the mucous membranes of the uterine cervical canal and the periphery of the external os in the pregnancy-confirmed-cows of day 18 after the artificial insemination was performed.

Example 2. Expression Analysis of Interferon Inducible Factor in Extrauterine Tissues (Uterine Cervical canal, Peripheral Portion of External Os of Uterus, and Vaginal Vestibule) of Pregnant Cows and Non-Pregnant Cows In the same way as in Example 1, except that a cotton swab was used as the collection instrument instead of the measuring spoon, mucous membranes were collected from the uterine cervical canal, the periphery of the external os of uterus, and the vaginal vestibule of pregnant cows, and from the uterine cervical canal in non-pregnant cows. Cells in the mucous membranes were collected by immersing the cotton swabs used for collection in ice-cooled 1.5 mL tubes filled with 500 µL of ISOGEN II (Nippon Gene). In the same way as in Example 1, the total RNAs were extracted, cDNAs were synthesized by the reverse transcription, and the real-time PCR was performed.

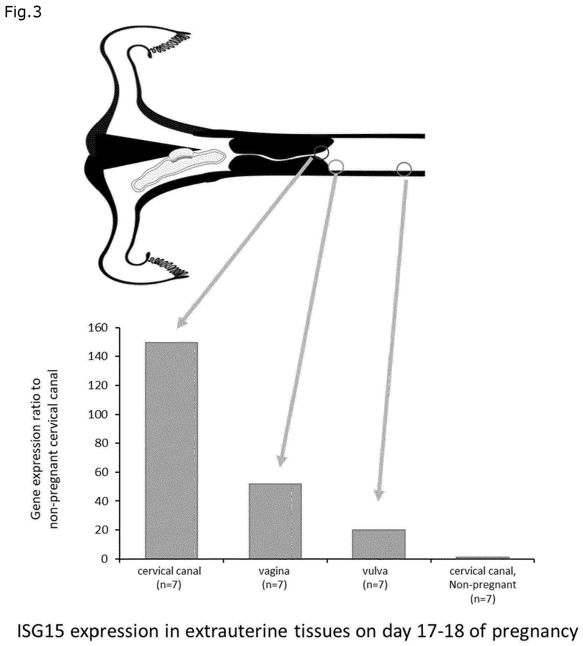

Fig. 3 illustrates the results of relative gene expression levels of ISG15. The expression of ISG15 was observed in all the mucous membranes of the uterine cervical canal, the periphery of the external os of uterus (deep portion of vagina), and the vulva (vaginal vestibule) during pregnancy. The gene expression levels were decreased with an increase in the distance from the uterus. However, compared with the expression level in the mucous membranes of the uterine cervical canal in the non-pregnant cows, the expression was increased by 20 to 150 times or more.

### Example 3. Expression Analysis of Interferon Inducible Factor in Extrauterine Tissues (Uterine Cervical canal) of Pregnant Cows and Non-Pregnant Cows by Quantitative RT-LAMP Method

The RNA samples derived from the mucous membranes of the uterine cervical canal in pregnant cows and non-pregnant cows collected and prepared using the same methods as those in 1) and 2) in Example 1 were adjusted their total RNA amount to 100 ng, and then contaminated DNA was digested by performing deoxyribonuclease (DNase) treatment on the samples. The RTLAMP reaction was directly performed using 100 ng of the RNA samples that have undergone DNase treatment derived from the mucous membranes of the uterine cervical canal in pregnant cows and non-pregnant cows, 100 ng of the RNA samples that have not undergone DNase treatment derived from the mucous membranes of the uterine cervical canal in pregnant cows and non-pregnant cows, and 1,000 ng of the RNA sample that has not undergone DNase treatment derived from the mucous membranes of the uterine cervical canal in non-pregnant cows.

The reaction conditions are as follows: 2.9 µL of sterilized distilled water, 12.5 µL of a reaction solution, 1 µL of an enzyme solution, LAMP primers including FIP (SEQ ID NO. 13): 40 pmol (0.8 µL), BIP (SEQ ID No. 14): 40 pmol (0.8 µL), F3 (SEQ ID No. 11): 5 pmol (1 µL), and B3 (SEQ ID No. 12): 5 pmol (1 µL) designed to amplify ISG15 were added per reaction, and 5 µL of the sample RNA solution was added to make a final 25 µL solution. The solution was reacted for 30 minutes and more at 65 degrees Celsius, and the turbidity of the reactant generated by the amplification of the target gene was measured by successive absorbance.

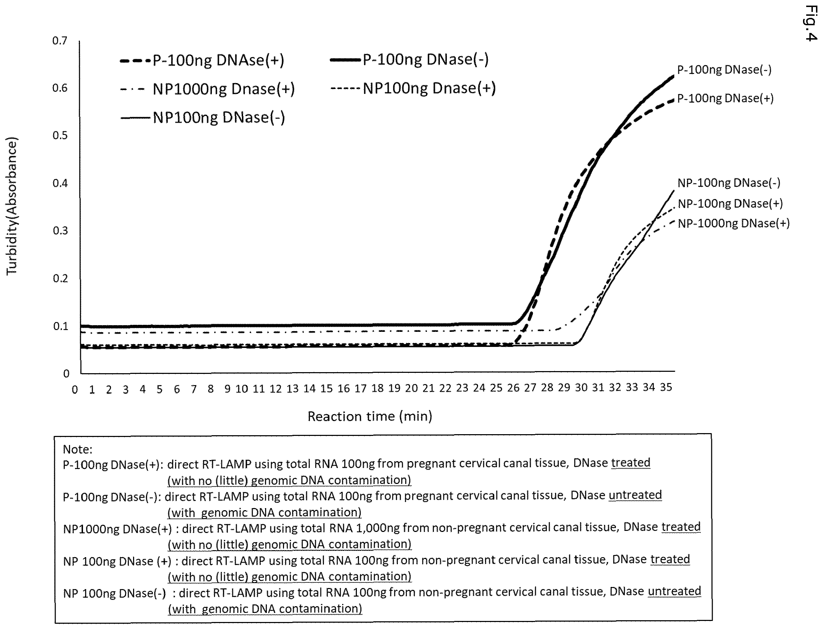

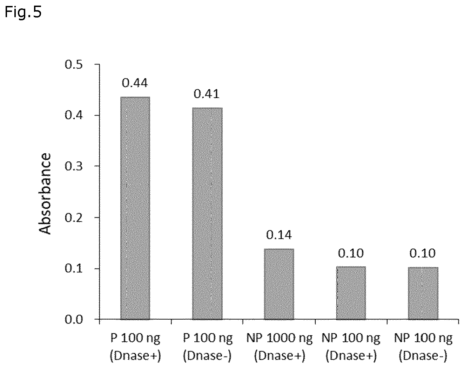

The results are shown in Fig. 4 and Fig. 5. Even when RNA was used as a template, it was possible to measure the expression level of ISG15. When the turbidities at 30 minutes after the start of reaction were compared, the expression level of ISG15 in the mucous membranes of the uterine cervical canal in pregnant cows was about four times as high as the expression level of ISG15 in the mucous membranes of the uterine cervical canal in non-pregnant cows. Moreover, the DNase treatment did not significantly affect the quantitative results. Even when the total RNA amount was increased by ten times, a significant change was not observed in the transition in turbidity. Consequently, it was confirmed that pregnancy could be determined even when the genomic DNA was contaminated.

## Claims

1. A method for determining pregnancy in a ruminant animal, the method comprising:
a measurement step of measuring an expression level of molecules induced in response to IFN-τ in mucosal epithelial cells, using a sample containing the mucosal epithelial cells, the sample being collected from the genital organs of a test ruminant animal ranging from the uterine cervical canal to the vulva in a period corresponding to the early stage of pregnancy; and
a determination step of determining pregnancy in the test ruminant animal by comparing the expression level with an expression level of the molecules in corresponding mucosal epithelial cells of a non-pregnant ruminant animal of the same species.

2. The method according to claim 1, wherein the sample containing the mucosal epithelial cells is collected from the genital organs ranging from the vagina to the vulva.

3. The method according to claim 1 or 2, wherein the sample containing the mucosal epithelial cells is collected from the surface of the genital organs in a low-invasive manner or in a non-invasive manner.

4. The method according to any one of claims 1 to 3, wherein the sample containing the mucosal epithelial cells does not substantially contain blood.

5. The method according to any one of claims 1 to 4, wherein each of the molecules induced in response to IFN-τ is mRNA transcribed from at least one gene selected from the group consisting of LOC100139670, ISG15, OAS-1X, OAS-1Y, OAS-1Z, OAS-2, UBA7, USP18, Mx1, Mx2, RSAD2, GBP4, GBP5, TNFSF10, STAT1, STAT2, and PLAC8 genes; or protein encoded from the gene.

6. The method according to any one of claims 1 to 5, wherein each of the molecules induced in response to IFN-τ is mRNA transcribed from at least one gene selected from the group consisting of ISG15, OAS-1X, Mx1, and Mx2 genes.

7. The method according to any one of claims 1 to 6, wherein the expression level of the molecules induced in response to IFN-τ is measured by a quantitative PCR method or a LAMP (loop-mediated isothermal amplification) method.

8. The method according to any one of claims 1 to 7, wherein the expression level of the molecules induced in response to IFN-τ is measured without separation and purification of RNA from the mucosal epithelial cells.

9. The method according to any one of claims 1 to 8, wherein the ruminant animal is selected from the group consisting of cow, sheep, goat, water buffalo, yak, and deer.

10. A kit for determining pregnancy in a ruminant animal, the kit comprising:
a reagent to measure an expression level of molecules induced in response to IFN-τ in mucosal epithelial cells of a test ruminant animal; and
an instrument to collect a sample containing the cells.

11. The kit according to claim 10, wherein each of the molecules induced in response to IFN-τ is mRNA transcribed from at least one gene selected from the group consisting of LOC100139670, ISG15, OAS-1X, OAS-1Y, OAS-1Z, OAS-2, UBA7, USP18, Mx1, Mx2, RSAD2, GBP4, GBP5, TNFSF10, STAT1, STAT2, and PLAC8 genes; or protein encoded from the gene.

12. The kit according to claim 10 or 11, wherein each of the molecules induced in response to IFN-τ is mRNA transcribed from at least one gene selected from the group consisting of ISG15, OAS-1X, Mx1, and Mx2 genes.

13. The kit according to any one of claims 10 to 12, wherein each of the molecules induced in response to IFN-τ is mRNA, and the reagent to measure the expression level of the molecules is a reagent for a nucleic acid amplification method.

14. The kit according to any one of claims 10 to 13, wherein the instrument to collect a sample containing the cells is an instrument to collect a sample in a low-invasive manner or in a non-invasive manner.


SEQUENCE LISTING

<110>  National University Corporation Hokkaido University

<120>  Method for Determining Pregnancy of Ruminants

<130>  P17HU03WO

<160>  14

<170>  PatentIn version 3.5

<210>  1
<211>  25
<212>  DNA
<213>  Artificial Sequence

<220>
<223>  Mx1 forward primer

<400>  1
gccaactagt cagcactaca ttgtc                                          25


<210>  2
<211>  23
<212>  DNA
<213>  Artificial Sequence

<220>
<223>  Mx1 Reverse primer

<400>  2
gctcttggac tccatatctt cac                                            23


<210>  3
<211>  20
<212>  DNA
<213>  Artificial Sequence

<220>
<223>  Mx2 forward primer

<400>  3
cagagacgcc tcagtcgaag                                                20


<210>  4
<211>  22
<212>  DNA
<213>  Artificial Sequence

<220>
<223>  Mx2 reverse primer

<400>  4
gagacgtttg ctggtttcca tg                                             22


<210>  5
<211>  20
<212>  DNA

<213>  Artificial Sequence

<220>
<223>  ISG15 forward primer

<400>  5
tgagggactc catgacggta                                                    20


<210>  6
<211>  20
<212>  DNA
<213>  Artificial Sequence

<220>
<223>  ISG15 reverse primer

<400>  6
gctggaaagc aggcacattg                                                    20


<210>  7
<211>  20
<212>  DNA
<213>  Artificial Sequence

<220>
<223>  OAS1X forward primer

<400>  7
tctgaggtcc agaaacggca                                                    20


<210>  8
<211>  23
<212>  DNA
<213>  Artificial Sequence

<220>
<223>  OAS1X reverse primer

<400>  8
agcttcacgt agatttgagg gtc                                                23


<210>  9
<211>  21
<212>  DNA
<213>  Artificial Sequence

<220>
<223>  H2AFZ forward primer

<400>  9
agagccggtt tgcagttccc g                                                  21


<210>  10
<211>  22
<212>  DNA
<213>  Artificial Sequence

<220>

<223>  H2AFZ reverse primer

<400>  10
tactccagga tggctgcgct gt                                                22


<210>  11
<211>  20
<212>  DNA
<213>  Artificial Sequence

<220>
<223>  LAMP F3 primer

<400>  11
agggactcca tgacggtatc                                                   20


<210>  12
<211>  19
<212>  DNA
<213>  Artificial Sequence

<220>
<223>  LAMP B3 primer

<400>  12
tgcttcagct ggacctcat                                                    19


<210>  13
<211>  41
<212>  DNA
<213>  Artificial Sequence

<220>
<223>  LAMP FIP

<400>  13
cacctccctg ctgtcaaggt ggagctgaag cagttcatcg c                           41


<210>  14
<211>  39
<212>  DNA
<213>  Artificial Sequence

<220>
<223>  LAMP BIP

<400>  14
ttgtcctcca gggcctgaga tcgttcctca ccaggatgg                              39